# EUROPEAN PATENT APPLICATION

(11) **EP 4 443 410 A1**
(43) Date of publication of application: **09.10.2024**
(21) Application number: 23167105.8
(22) Date of filing: 06.04.2023
(51) Int. Cl.: G09B 5/12, G09B 19/00

(54) **SYSTEM AND METHOD FOR ERGONOMIC TRAINING**

(71) Applicant: MotionMiners GmbH, 44227 Dortmund (DE); Fraunhofer Austria Research GmbH, 1040 Wien (AT)
(72) Inventor: GRZESZICK, René, 44227 Dortmund (DE); REINOLD, René, 44139 Dortmund (DE); STACH, Maximilian, 44225 Dortmund (DE); RIESTER, Martin Alexander, 1030 Wien (AT); HAYEK, Matthias, 2320 Schwechat (AT)
(74) Representative: Pfenning, Meinig & Partner mbB

(57) **Abstract**

The invention relates to a system for ergonomic training, comprising a server, a plurality of stations, each of the stations including at least one predefined object to be handled by a test person, wherein a predefined manner of handling the predefined object is defined for each predefined object, and each station comprising a localization arrangement. The system furthermore comprises a mobile kit to be carried by a test person, including at least three wearable inertial sensors to be worn by the test person and configured for recording and storing sensor data indicative of a movement of the test person, wherein the mobile kit further includes a localization device configured for interaction with the localization arrangement of each station. The system is configured to determine that the localization device is in proximity of the localization arrangement of a specific station, based on an interaction between the localization device and the localization arrangement of the specific station. The invention also relates to a method for ergonomic training.

## Description

The present invention is in the field of ergonomic training. It may advantageously be used in the context of order picking.

EP 3 454 744 A1 shows systems and devices for motion tracking, assessment, and monitoring and methods of use thereof.

US 2021/0290176 A1 shows systems and methods for improving workplace safety.

US 2021/0389817 A1 shows methods and apparatuses for actions, activities and task classifications based on machine learning techniques.

It is an object of the present invention to provide for at least one of the following aspects:
- providing a versatile and customizable solution for ergonomic training,
- increasing reliability of obtained data and information derived therefrom,
- creating a simple and portable setup,
- improving user experience and/or learning impact.

This is achieved by a system for ergonomic training as defined in claim 1. It is also achieved by a method according to the co-ordinate claim. Advantageous embodiments are shown in the dependent claims and in the following description and the figures.

A system for ergonomic training, comprises a server.

The system furthermore comprises a plurality of stations, each station including at least one predefined object to be handled by a test person, wherein a predefined manner of handling the predefined object is defined for each predefined object. Moreover, each station comprises a localization arrangement. Each station may comprise one or more localization arrangements, which may for instance be provided on one or more of the predefined objects and/or separate from the predefined objects.

The system furthermore comprises a mobile kit to be carried by a test person. The mobile kit includes at least three wearable inertial sensors to be worn by the test person and configured for recording and storing sensor data indicative of a movement of the test person. The mobile kit further includes a localization device configured for interaction with the localization arrangement(s) of each station.

The system is configured to determine that the localization device is in proximity of the localization arrangement of a specific station, based on an interaction between the localization device and the localization arrangement of the specific station.

The system is furthermore configured for storing information enabling identification of the specific station, in response to determining the proximity between the localization arrangement of the specific station and the localization device, and for storing subsequently recorded sensor data in a manner associable with the information enabling identification of the specific station. The server is configured for receiving the information enabling identification of the specific station and the sensor data associable with it.

The server is configured for retrieving information on the at least one predefined object and on the predefined manner of handling the at least one predefined object for the specific station.

The server comprises a machine learning algorithm configured to process the sensor data, along with the information on the at least one predefined object and on the predefined manner of handling the at least one predefined object, in order to evaluate a quality of movements carried out by the test person when handling the at least one predefined object in the predefined manner.

A method for ergonomic training according to the present application may comprise the following steps:
A step of providing a plurality of stations, each station including at least one predefined object to be handled by a test person, wherein a predefined manner of handling the predefined object is defined for each predefined object, and each station comprising a localization arrangement.

A step of equipping a test person with a mobile kit, the mobile kit including at least three wearable inertial sensors to be worn by the test person and configured for recording and storing sensor data indicative of a movement of the test person, the mobile kit further including a localization device configured for interaction with the localization arrangement of each station.

A step of providing instructions to the test person to handle at least a subset of the at least one predefined object of at least a subset of the plurality of stations in the respective predefined manner.

Within the method, test person may move consecutively to each of the stations of the subset of the plurality of stations. There, the test person handles the subset of the at least one predefined object at each of these stations in the respective predefined manner.

Moreover, the following further steps may be executed:
An interaction between the localization device and the localization arrangement of a specific station is detected. Based on the detected interaction, it is determined that the localization device is in proximity of the localization arrangement of a specific station.

Information enabling identification of the specific station is stored, in response to determining the proximity between the localization arrangement of the specific station and the localization device. Subsequently, sensor data is recorded in a manner associable with the information enabling identification of the specific station.

The information enabling identification of the specific station and the sensor data associable with it are uploaded to a server.

Information on each specific object pertaining to the subset of the at least one predefined object is retrieved. Additionally, information on the predefined manner of handling each specific object is retrieved.

A machine learning algorithm is employed for processing the sensor data, along with the information on each specific object, and along with the information on the predefined manner of handling each specific object, in order to evaluate a quality of movements carried out by the test person when handling each specific object in the predefined manner.

The machine learning algorithm may in particular include an algorithm for human activity recognition. This human activity recognition may be employed in order to identify a specific human activity when the object is being handled. Based on this identification, the quality of the movements may be evaluated in a particularly targeted way. This will be explained further below.

According to the present application, a method may include using the system shown and described herein. In particular, the above-described method may employ some or all of the system components shown and described herein. It should be noted that features described herein with respect to the system may also be claimed in conjunction with the method. Vice versa, aspects explained in the context of the method may also be claimed in conjunction with the device. In particular, components of the system may be specifically configured to facilitate and/or carry out steps or aspects of the method.

The device and method enable a versatile assessment of ergonomics. Therein, they may be dynamically employed and adjusted for use in a specific environment found at a client's or user's location or facilities. The test person can then complete a course that can be individually set up, and is not subject to any constraints in terms of size or spatial extent. The environment does typically not need to be altered, and there is no need to install cameras or other surveillance equipment.

While generally not being limited in size, the system may for instance comprises at least 5 stations or at least 20 stations. For instance, it may comprise 30 stations.

At each of the plurality of stations, for example, at least two or at least three predefined objects may be provided, and among those, any subset may be handled by the test person. The various objects may be identical to each other in terms of size and/or weight, or they may differ from each other in terms of size and/or weight.

The subset may be chosen based on a specific aim of the test to be performed and for instance in accordance with the test person's work description. For example, the test person may perform 2-3 picks per station, performing different kinds of movements with different kinds of objects.

If there are several predefined objects at a given station, they may be handled in the same manner, or in a manner different from each other. For example, the type of object and their size and shape may be the same for all objects within one station, and they may be different for objects included in another station. In another example, the objects may differ from each other within a single station.

These different ways and combinations of setting up the system and carrying out the method provide flexibility in which types of movements can be assessed and in how the system may be integrated into a given location or environment.

In an example, the various predefined objects may include predefined objects that differ from each other in weight and/or size. Then, the server may be configured for retrieving information on weight and/or size of each predefined object and for including this information in the evaluation of the quality of movements carried out by the test person when handling the respective predefined object in the predefined manner.

The device and method advantageously facilitate taking into account characteristics of the predefined objects in order to evaluate the movements, since the information of the predefined objects is retrieved and may be used when assessing whether or not handling of the object has been performed in an ergonomically advantageous way. For example, the information on the predefined object may include information on size and/or weight. By taking these aspects into account, an unergonomic or harmful movement performed with a heavier object may be assigned a stronger negative rating than an unergonomic or harmful movement performed with a light object. For example, a heavier object may require a different posture than a light object, and this may readily be taken into account here. Also, problematic postures can be evaluated according to whether they are due to the dimensions of an unmanageable object or whether they are due to unnecessary and therefore avoidable movements of the test person.

For example, the predefined manner of handling the predefined object may include, at at least some of the stations, one or more of the following actions: picking the object, lifting up the object, removing the object from a shelf, putting down the object, inserting the object into a shelf, carrying the object over a distance, moving the object by way of a hand cart. For example, inserting the object into a shelf may include inserting the object into the shelf at a predefined height, and/or inserting several objects at various different heights.

For example, evaluation of the quality of the movements may include identification of one or more of the following actions performed by the test person, based on the sensor data: walking, standing, driving a vehicle, handling a hand cart, handling an object using one hand, handling an object using two hands, lifting an object, holding an object, carrying an object, bending a back of the test person, torsion of the back of the test person, handling an object in front of a body of the test person, handling an object above a shoulder of the test person. These actions may be identified by way of the human activity recognition of the machine learning algorithm. Subsequently, the quality of these activities and of the movements performed during these activities can be evaluated in an advantageous manner.

To this end, the system may be configured to determine classes of movement associated with the above-listed actions. The at least three wearable inertial sensors gather relevant information for enabling identification of these classes of movement. The server employs machine learning to determine classes of movement and/or posture, based on the received sensor data. From this, the above-listed actions may for instance be derived and their quality and ergonomicity can subsequently be determined.

The at least three wearable inertial sensors include a first wearable inertial sensor that is included in a waist- or hip-belt. Additionally and/or alternatively, the at least three wearable inertial sensors may comprise a second wearable inertial sensor that is included in a wrist band and, for example, a third wearable inertial sensor that is included in a further wrist band. The test person may carry the belt with the first inertial sensor around their waist or hip and may carry the wrist bands with the second and third inertial sensors around their wrists, i.e., one at the left wrist and one at the right wrist.

In an embodiment, the system comprises, for each test person, precisely the three above-mentioned types of wearable inertial sensors, i.e., the first wearable inertial sensor that is included in the waist- or hip-belt, the second wearable inertial sensor that is included in the wrist band, and the third wearable inertial sensor that is included in the further wrist band, and no further sensors are envisioned for a given test person. Of course, several sets of sensors may be provided, so that more than one test person can employ the system at the same time. It was found that provision of these three sensors represents an advantageous setup that enables capturing motions as required for the method and system, while keeping the system and data processing required comparably slim and simple. In particular, this setup enables human activity recognition in an advantageous manner.

In an example, the wearable inertial sensors include an accelerometer and/or a gyroscope and/or a magnetometer. For example, each of the three wearable inertial sensors may include one or more of an accelerometer and/or a gyroscope and/or a magnetometer.

For example, a recording rate of the sensors may be at least 33 Hz. In another example, the recording rate of the sensors is at least 50 Hz. For example, the recording rate may be 100 Hz.

In an example of carrying out the system or method, the mobile kit may comprise a mobile electronic device (MED). The MED may be carried out as a Portable Data Terminal (PDT) and/or a smartphone and/or a portable computer.

In an example, the mobile electronic device may include the localization device of the mobile kit. However, the localization device may for instance also be included in the wearables of the wearable inertial sensors. The localization device may also be provided separate from the inertial sensors and a possible mobile electronic device.

In an example, the mobile electronic device may comprise an output device for providing instructions to the test person.

In an example, the localization arrangement(s) of the station(s) may include a first wireless communication device, and the localization device of the mobile kit may comprise a second wireless communication device, configured for interaction with the first wireless communication device. The first and/or second wireless communication devices may for instance be carried out as WiFi-devices, Ultra-Wideband-Devices, Radio-Wave-Devices. In an example, the first wireless communication device may provide a beacon, such as a Bluetooth beacon.

Additionally or alternatively, the localization arrangement may comprise a scannable code, such as a barcode or a QR-Code, and the localization device may comprise a scanner for scanning the scannable code. Proximity may then be determined, since it is necessary for the test person to be close to the code, in order to scan the code.

Each station may comprise one or more localization arrangements. They may be provided on one or more of the objects of a given station, for example. They may however also be provided separate from the objects. In an example, a scannable code may be provided on each of the objects. This may facilitate providing instructions relating to a specific scanned object to the test person and/or retrieving information on a given object during the evaluation carried out on the server.

In an example of the system or method, the mobile kit may include at least one memory for storing the information enabling identification of the specific station and the sensor data associable with it. For example, the at least one memory may include a memory in some or in each one of the at least three wearable sensors. For example, a separate memory may be provided for each of the wearable inertial sensors, or a joint memory may be provided, communicatively connected to all of the wearable inertial sensors worn by the test person. Additionally or alternatively, the mobile kit may comprise a memory in the mobile electronic device.

The mobile kit may include a communication system, in particular a wireless communication system, configured for communication between the mobile electronic device and the at least three wearable sensors.

This communication system enables interaction between the components of the mobile kit, for instance in order to transfer information on a detected proximity, as detected by the mobile electronic device, to the at least three wearable sensors, in order to enable storing sensor data in a manner associable with the detected proximity.

For example, in an example where the localization device is provided in the mobile electronic device, data relating to its interaction with a localization arrangement of a station may be transferred to the wearable sensors. The wearable sensors may comprise one or more memories, and the data relating to the interaction with a localization arrangement may be saved in these memories along with the sensor data that is recorded in a manner temporally related to the detected interaction with the localization arrangement. The detected interaction with the localization arrangement may enable an identification of a specific station, and of a specific object at that station that is supposed to be handled by the test person at the station, and of a specific manner in which the object is supposed to be handled. This information may then be paired with the sensor data to evaluate quality of movements.

In an example, the system may comprise a docking station for at least some of the components of the mobile kit. The docking station may be connected to the server and enable upload of the information enabling identification of the specific station and the sensor data associable with it from the memory to the server. For example, data upload to the server from or through the docking station may be done using WiFi and/or an internet connection, enabling providing the system away from the server, at any desired location.

For example, the docking station may be configured to receive the wearable sensors. In an example, the memories of the wearable sensors hold the necessary data for evaluating the movements, wherein data from an optional mobile electronic is communicated to the wearable sensors before docking. This way, it is sufficient to dock the wearable inertial sensors, and the mobile electronic device does not need to be docked. Vice versa, it is also possible to have a memory in the mobile electronic device, and transferring the sensor data from the wearable sensors to the mobile electronic device, and then establishing a data transfer from the mobile electronic device to the server.

In another example, it may also be envisioned to establish a direct communication with the server, for the wearable inertial sensors and/or for the optional mobile electronic device, wherein data may be uploaded without use of a docking station.

In possible embodiments of the system and method, it may be envisioned that a supervisor oversees the ergonomic training. Optional ways of carrying out the system include an interaction device for interaction with the supervisor, in particular to enable input by the supervisor. The system may be designed to enable the supervisor to provide an additional assessment or evaluation of the movements carried out by the test person. Additionally or alternatively, the system may be designed to enable the supervisor to design and/or alter a training course.

In an example, the system includes an input device for a supervisor, configured for providing one or more input options to allow the supervisor to input an assessment of a quality of predefined aspects of one or more movements carried out by the test per-on when handling the predefined object in the predefined manner. The server may be configured for receiving the input by the supervisor, and for including said input by the supervisor into the evaluation of the quality of movements carried out by the test person when handling the object in the predefined manner. For example, supervisor input can be provided for each station, or it can be provided jointly for all stations, for example after the test person has terminated all stations. An ergonomic score can be computed that comprises the sensor data and the input from the supervisor, in addition to the information on the predefined objects and the predefined manners of handling the predefined objects.

Input options given to the supervisor may include a requested evaluation of movements, in particular small movements, that are not or not fully captured by the wearable inertial sensors.

In an example of the system or method, the one or more input options for the supervisor include an input option for one or more of
- assessing a movement or a posture of a shoulder of the test person, assessing a movement or a posture of a back of the test person,
- assessing a movement or a posture of a leg or of legs of the test person,
- assessing a movement or a posture of an arm or of arms of the test person,
- assessing a movement or a posture of a hand or of hands of the test person.

Input option for the supervisor may be provided for assessing a combined movement of the above-described body parts, such as a combined movement of a combination of at least two of a shoulder, a back, a leg, an arm, a hand, in order to provide an evaluation of whether these body parts are being kept in an advantageous or in a potentially damaging way with respect to each other.

It may also be envisioned to provide input options for the supervisor to specify environment conditions.

In an example of the system or method, the input device for the supervisor may provide an input option for selecting a subset of the plurality of stations by the supervisor and/or for selecting a subset of predefined objects of a given station by the supervisor.

In an example, the input device for the supervisor is connected to the internet, for instance by WiFi, and the input options for the supervisor are provided in a web-interface.

In an example, the method includes:
A step of providing an input device to a supervisor.

A step of providing, on the input device, one or more input options to allow the supervisor to input an assessment of a quality of predefined aspects of one or more movements carried out by the test person when handling the predefined object in the predefined manner.

A step of transmitting the supervisor's input to the server, and including said input into the evaluation of the quality of movements carried out by the test person when handling the object in the predefined manner. The supervisor's input may be provided and/or stored in a manner associable with specific stations and/or objects at these stations and/or the predefined manner of handling these objects.

The invention will now be explained in an exemplary manner with reference to the appended figures.

Therein:
- Fig. 1a: shows a schematic setup of a system for ergonomic training,
- Fig. 1b: shows components of the system for ergonomic training, in an example including a scannable code,
- Fig. 1c: shows components of the system for ergonomic training, in an example including a Bluetooth beacon,
- Fig. 2a: shows a mobile electronic device of the system for ergonomic training, to be used by a test person,
- Fig. 2b: shows a set of wearable inertial sensors of the system for ergonomic training, to be worn by the test person,
- Fig. 2c: shows a docking station for the wearable inertial sensors,
- Figs. 3a-d: show a station of the system for ergonomic training and illustrate steps performed by the test person at this station,
- Fig. 4: shows a further station of the system for ergonomic training, comprising different predefined objects to be handled by the test person,
- Fig. 5: illustrates steps performed in a method for ergonomic training, employing the system for ergonomic training,
- Figs. 6a-b: illustrate association of sensor data of the wearable inertial sensors with data indicative of a proximity of a mobile electronic device to a specific station.

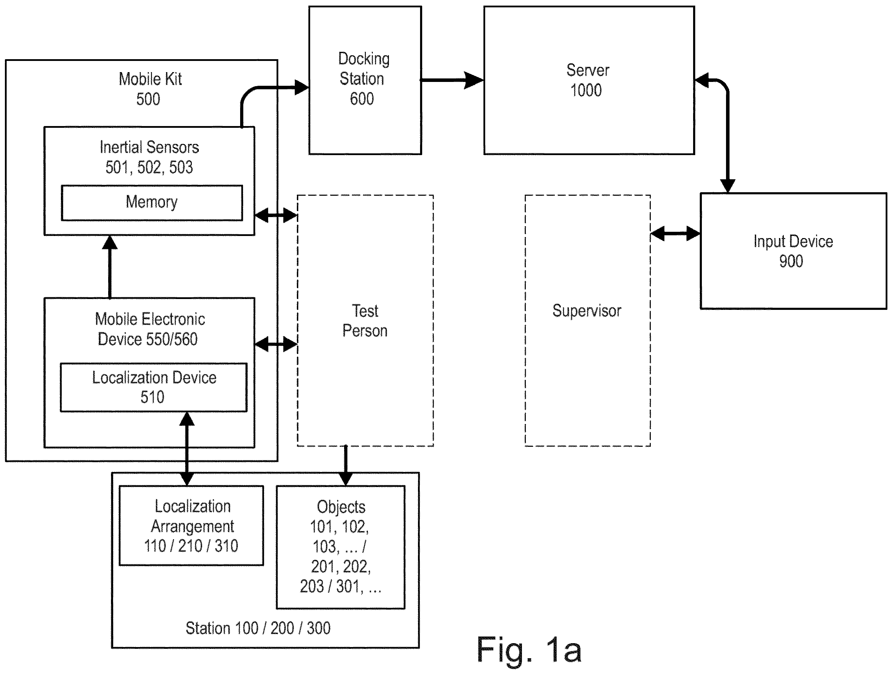

Fig. 1a shows a schematic setup of a system for ergonomic training, which may be used for carrying out a method for ergonomic training.

The system comprises a plurality of stations 100, 200, 300, each of which includes at least one predefined object 101, 102, 103, 201, 202, 203, 301 to be handled by a test person performing the ergonomic training. A predefined manner of handling the predefined object 101, 102, 103, 201, 202, 203, 301 is defined for each predefined object 101, 102, 103, 201, 202, 203, 301. Within the method, ergonomicity of the movements of the test person when handling the objects is evaluated, and the results of the evaluation may be presented to the test person, so that the test person can improve their movement patterns, posture, and the like, and, for example, overcome bad habits in this regard. By providing several stations with several objects, an abundance of different movements may be monitored and assessed, as will be further explained here below. This may provide a particularly broad and accurate picture of the movements of each test person. The system is particularly advantageous in that it may be used in a flexible and versatile manner for a variety of different stations and movements, and it may be used in different places. Specifically, modifying or altering the stations and their objects can be done essentially without requiring modification of the remaining components of the system. These aspects will become more apparent from the explanations given in conjunction with the further figures.

Each station 100, 200, 300 comprises a localization arrangement 110, 210, 310, which enables detection of whether the test person is in the vicinity of the respective station.

The system furthermore comprises a mobile kit 500 to be carried by the test person. The mobile kit 500 includes at least three wearable inertial sensors 501, 502, 503 to be worn by the test person and configured for recording and storing sensor data indicative of a movement of the test person. To this end, one or memories are included in the at least three wearable inertial sensors.

The mobile kit 500 further includes a localization device 510 configured for interaction with the localization arrangement 110, 210, 310 of each station 100, 200, 300. In the example of Fig. 1a, the localization device 510 is included in a mobile electronic device 550/560, which the test person carries as they perform the training.

The system is configured to determine that the localization device 510 is in proximity of the localization arrangement 110, 210, 310 of a specific station 100, 200, 300, based on an interaction between the localization device 510 and the localization arrangement 110, 210, 310 of the specific station.

If such an interaction is detected and consequently proximity to a specific station is determined, the mobile electronic device interacts with the wearable inertial sensors, to trigger recording of the sensor data, and to submit information on the specific station to the wearable inertial sensors, which are configured to store this information in their memory or memories. To this end, the mobile kit 500 comprises a communication system for communication between the mobile electronic device and the wearable inertial sensors. Consequently, the system is configured for storing information enabling identification of the specific station 100, 200, 300, in response to determining the proximity between the localization arrangement 110, 210, 310 of the specific station and the localization device 510, and for storing subsequently recorded sensor data in a manner associable with the information enabling identification of the specific station 100, 200, 300. While the test person is performing the training, all the relevant sensor data and associated information enabling identification of the station at which the sensor data was recorded, may be kept in the memory or memories of the inertial sensors.

Evaluation of the movements carried out by the test person is subsequently performed on a server 1000, which comprises a machine learning algorithm.

The server 1000 is configured for receiving the information enabling identification of the specific station 100, 200, 300 and the sensor data associable with it. Here, this is done via a docking station 600 where the inertial sensors may be docked after all stations of the training course have been completed by the test person. The docking station 600 is communicatively connected to the server 1000 and the relevant data from the memory or memories of the inertial sensors may be uploaded to the server using WiFi, for example.

The server 1000, on the other hand, receives this information and is configured for retrieving information on the at least one predefined object 101, 102, 201, 202, 301, 302 and on the predefined manner of handling the at least one predefined object 101, 102, 103, 201, 202, 203, 301 for each specific station where an action was performed by the test person.

The server 1000 employs the aforementioned machine learning algorithm in order to process the sensor data, along with the information on the at least one predefined object 101, 102, 103, 201, 202, 203, 301 and on the predefined manner of handling the at least one predefined object 101, 102, 103, 201, 202, 203, 301, in order to evaluate the quality of movements carried out by the test person when handling the at least one predefined object 101, 102, 103, 201, 202, 203, 301 in the predefined manner.

The system in the example of Fig. 1a further includes an optional input device 900 for a supervisor. The supervisor may interact with the server 1000 via this device 900, for example to design a specific course for the test person according to his or her needs, but also to submit further evaluations of the test person's movement, to create an even more exhaustive picture of ergonomicity of their movements. To this end, the input device 900 is configured for providing one or more input options to allow the supervisor to input an assessment of a quality of predefined aspects of one or more movements carried out by the test person when handling the predefined object 101, 102, 103, 201, 202, 203, 301 in the predefined manner.

The server 1000 is configured for receiving the input by the supervisor, and for including said input by the supervisor into the evaluation of the quality of movements carried out by the test person when handling the object 101, 102, 103, 201, 202, 203, 301 in the predefined manner.

The input device 900 for the supervisor furthermore provides an input option for selecting a subset of the plurality of stations 100, 200, 300 by the supervisor and for selecting a subset of predefined objects of each station by the supervisor. The input device 900 for the supervisor is connected to the internet, and the input options for the supervisor are provided in a web-interface.

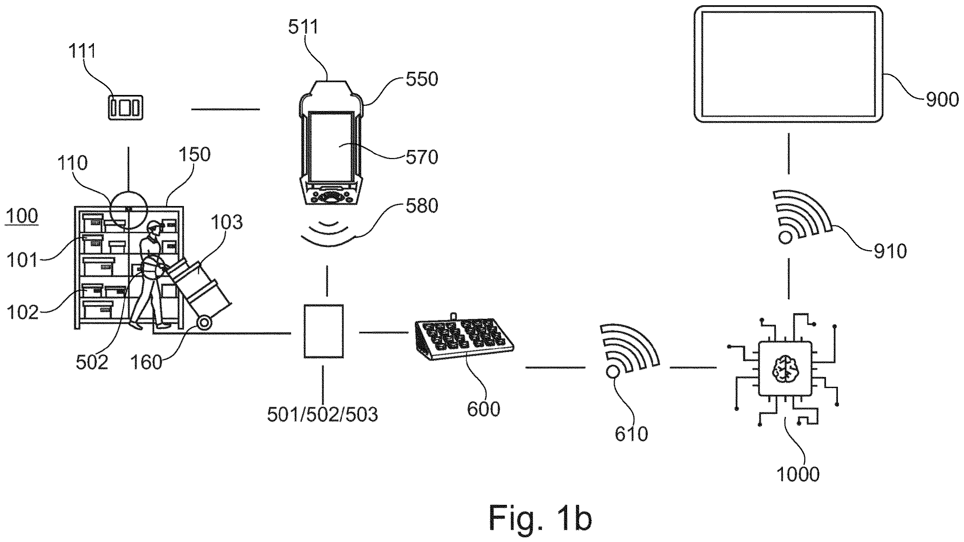

Fig. 1b shows components of the system for ergonomic training, in an example including a scannable code. Exemplarily, a single station 100 is shown, wherein the system typically comprises further stations. All functions explained in conjunction with Fig. 1a may apply for the system of Fig. 1b.

The station 100 includes the localization arrangement 110, which comprises a scannable code 111 that is exemplarily carried out as a barcode.

The station 100 includes several predefined objects 101, 102, 103 of different weights, sizes and shapes, that are initially provided in a shelf 150. The station 100 additionally includes a hand cart 160.

The mobile electronic device of the mobile kit 500 is here a Portable Data Terminal 550, which includes the localization device 510 in the form of a scanner 511 for scanning the scannable code 111. Moreover, the Portable Data Terminal 550 has an output device 570 in the form of a screen and a wireless communication system 580 for providing instructions to the test person, which may help lead the test person through the course of stations.

When the test person arrives at the station 100, they scan the scannable code 111 using the mobile electronic device. From this, it is clear that the test person is in the vicinity of the specific station 100, because they must be close to the scannable code 111 in order to scan it.

Instructions may be displayed to the test person on the output device 570 of the Portable Data Terminal 550, indicating which of the objects 101, 102, 103 they should manipulate and in which way. Exemplarily, the test person may be instructed to remove object 103 and further objects from the shelf, put them on the hand cart 160 and push the hand cart 160 to a predetermined location. In order to enable provision of such instructions via the portable data terminal, it may be communicatively connected to the server 1000, where the instructions may be stored (and potentially altered by the supervisor), or it may hold these instructions in an internal storage.

Moreover, scanning of the code triggers an interaction between the mobile electronic device 550 and the wearable inertial sensors 501, 502, 503, via the communication system 580, and the wearable inertial sensors may start recording subsequent to this interaction. I.e., in response to the detected interaction, the mobile kit 500 stores the information enabling identification of the specific station and the sensor data associable with it to the memory of the inertial sensors 501, 502, 503.

After the course has been completed, the test person docks the wearable inertial sensors in the docking station 600, which is connected to the server 1000 and enables upload of the information enabling identification of the specific station and the sensor data associable with it from the memories of the wearable inertial sensors 501, 502, 503 to the server 1000.

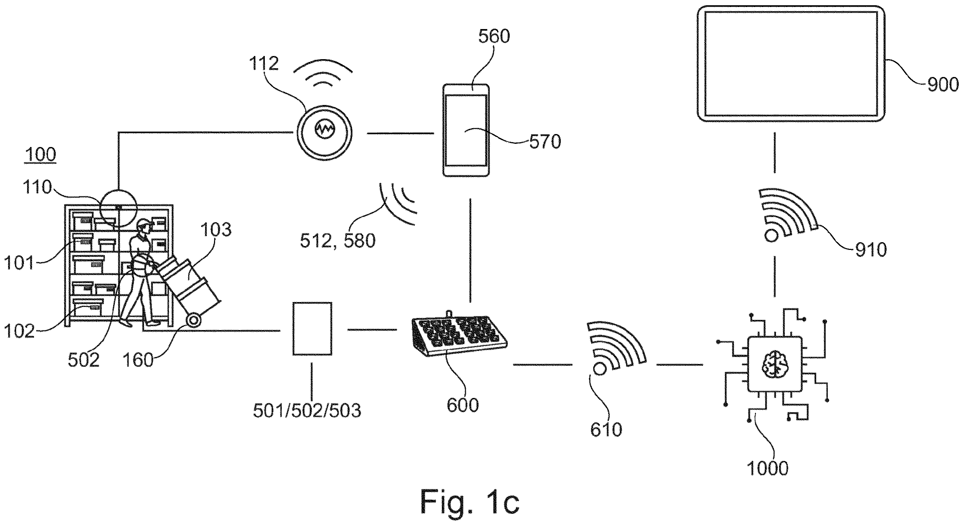

Fig. 1c shows components of the system for ergonomic training, in an example including a Bluetooth beacon, which may be included in addition or as an alternative to the scannable code of Fig. 1b. All functions explained in conjunction with Figs. 1a and 1b may apply for the system of Fig. 1c.

The localization arrangement 110 of the station includes a first wireless communication device 112, which functions as a Bluetooth beacon.

The mobile kit 500 comprises a mobile electronic that is a smartphone 560. It includes the localization device 510, which comprises a second wireless communication device 512, which is a Bluetooth device configured for interaction with the first wireless communication device 112. The display of the smartphone is used for providing instructions the test person and leading them from one station to the next.

In this case, proximity of the test person to the station 100 may readily be detected via the Bluetooth beacon, and the further step starting the measurements may be initiated based thereon, without requiring the test person perform a scan.

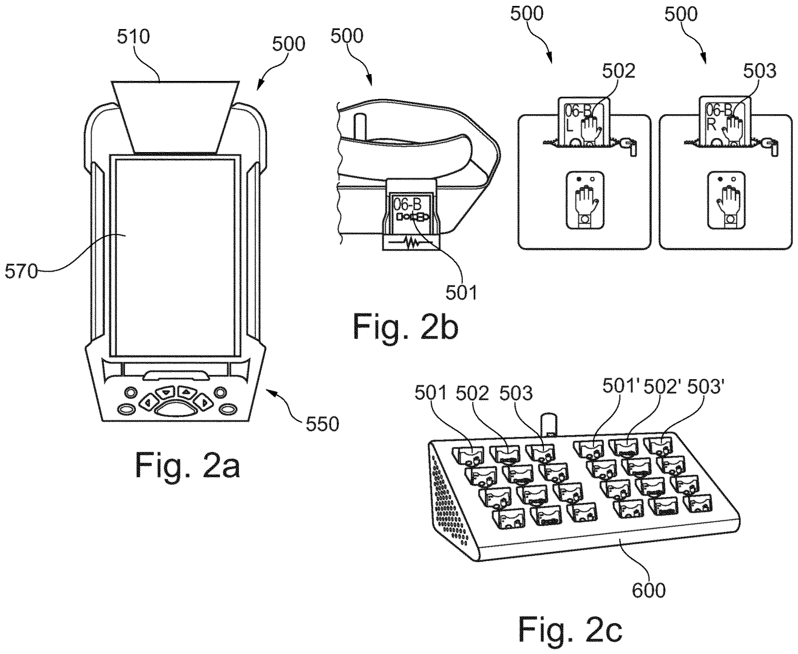

Figs. 2a and 2b show the components of the mobile kit 500. Fig. 2a shows the mobile electronic device, which comprises the localization device 510 in the form of a scanner and the output device 570 in the form of a display.

Fig. 2b shows a set of wearable inertial sensors 501, 502, 503 of the system for ergonomic training, to be worn by the test person. These are a total of exactly three wearable inertial sensors 501, 502, 503 for each test person, including a first wearable inertial sensor 501 that is included in a waist- or hip-belt and a second wearable inertial sensor 502 that is included in a wrist band and a third wearable inertial sensor 503 that is included in a further wrist band.

The wearable inertial sensors 501, 502, 503 each include an accelerometer and/or a gyroscope and/or a magnetometer.

Their recording rate of the inertial sensors is 100 Hz.

Fig. 2c shows a docking station 600, wherein eight sets of wearable inertial sensors are docked. Two of the sets are exemplarily marked with reference signs 501, 502, 503, 501', 502', 503'. Accordingly, there are exactly three wearable sensors per person, and eight people may perform the ergonomic training simultaneously. The docking station 600 comprises a WiFi-system for communication with the server 1000. The docking station 600 is configured to charge the wearable inertial sensors while they are docked.

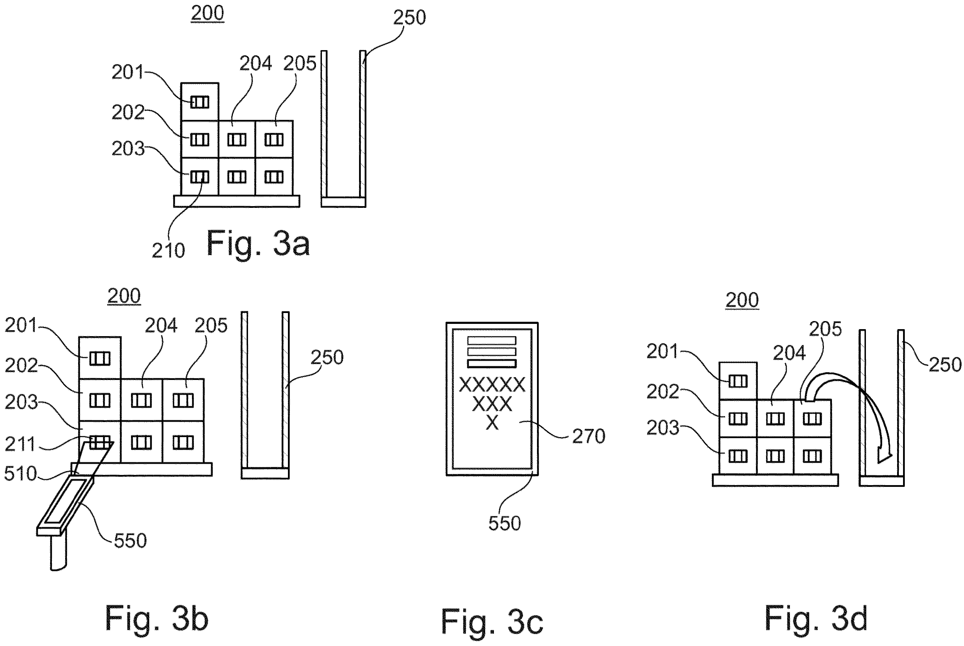

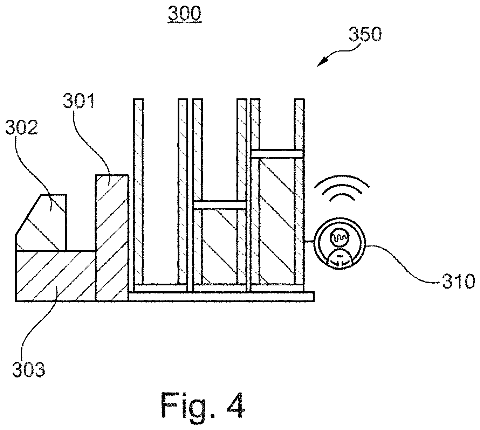

Figs. 3a-d and 4 relate to further stations 200, 300, which represent further examples for carrying out the stations of the system. They comprise different predefined objects to be handled by the test person. The system may for instance comprise between 5 and 50 stations, which may be carried out similar or identical to station 100 as shown in Figure 1b or 1c, or similar or identical to the stations 200 and 300 of Figures 3a or 4.

Turning to Figures 3a-d, station 200 includes seven predefined objects (only some of which are provided with reference sings 201, 202, 203, 204, 205). Initially, some of the predefined objects 201, 202, 203, 204, 205 are stacked on top of each other, as shown in Fig. 3a. They all have a similar box-shape, but they may for instance differ from each other in weight. Moreover, they differ from each other in their initial position (i.e. the height at which they are arranged) and consequently, if they are to be handled by the test person, the manner of handling will be different, depending on whether they need to be retrieved from atop another object, or from the ground, for example. Each object is specifically identifiable and the parameters of each object, such as their initial position and weight are known (i.e., predefined) and stored on the server 1000. This information will be included in the evaluation of the quality of movements. Each of the objects comprises a localization arrangement in the form of a bar code.

Next to the stacked objects, a shelf 250 is provided. The test person will be required to move at least some of the stacked objects into the shelf. Depending on how many boxes are already in the shelf, the movement of placing the objects will be different. This is also taken into account by the evaluation performed on the server.

When the test person arrives at the station, they will scan the barcode 211 on one of the objects (Fig. 3b), using the mobile electronic device 550. Scanning the barcode 211 is an interaction between the mobile electronic device 550 and the station 200 from which it can be derived that the test person is now at station 200. Subsequently, instructions will be displayed on the screen 570 of the mobile electronic device 550, instructing the test person to handle one or more of the objects 201, 202, 203, 204, 205 in a predefined manner and a predefined order (Fig. 3c). These instructions include one or more of picking up one of the objects 201, 202, 203, 204, 205, lifting up one of the objects 201, 202, 203, 204, 205, putting down one of the objects 201, 202, 203, 204, 205, inserting one of the objects 201, 202, 203, 204, 205 into the shelf 250, removing one of the objects 201, 202, 203, 204, 205 from the shelf 250, carrying one of the objects 201, 202, 203, 204, 205 over a distance, moving one of the objects 201, 202, 203, 204, 205 by way of a hand cart. For example, the test person may be required to first place object 205 on the bottom of the shelf 250 (Fig. 3d). Also, subsequently to the detected interaction, the wearable inertial sensors will initiate recording sensor data which is thus associable to the detected interaction (for example by way of a time stamp) and which makes it thus possible to assign this specific set of sensor data to the specific object 205 and to the specific type of movement of placing an object from a medium height onto ground level, for example.

Fig. 4 shows a further station 300, which includes three different objects 301, 302, 303, that differ considerably from each other in size and shape. They may also differ from each other in weight. Additionally, station 300 comprises a shelf or rack 350 with three compartments having boards at different heights. The station 300 is equipped with a localization arrangement 310 in the form of a Bluetooth beacon. Once the test person enters the vicinity of the station 300, the localization device 510 of their mobile kit 500 will detect this vicinity. Detection of proximity, again, triggers displaying instructions to the test person, and recording of the sensor data so that it can be paired with the test person carrying out the instructions.

It is understood that the features shown and described for stations 100, 200 and 300 may be mixed and matched according to the needs of the specific training.

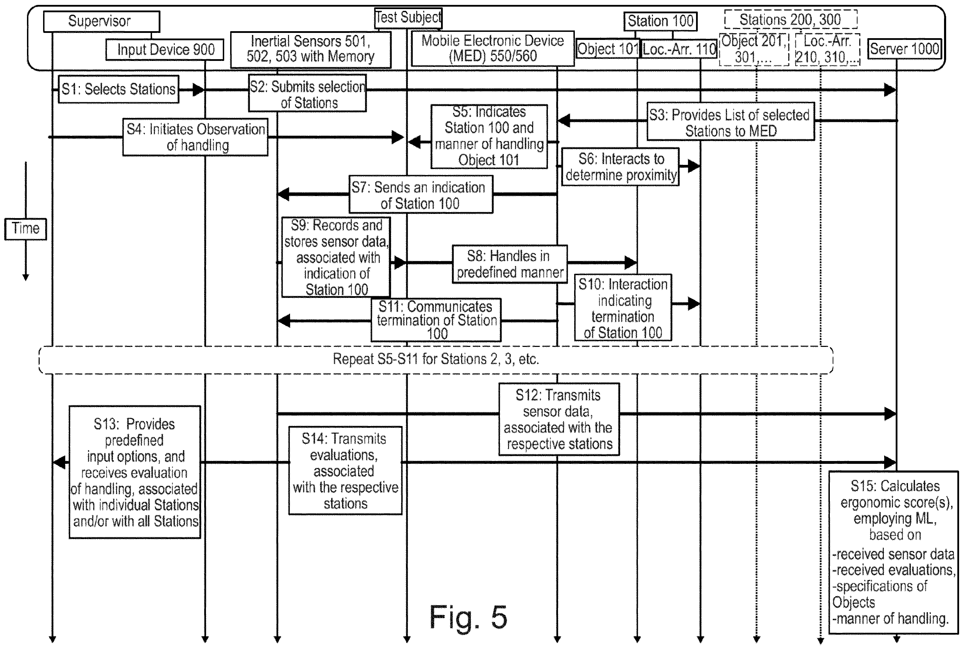

Fig. 5 illustrates steps performed in a method for ergonomic training, employing the system for ergonomic training, in particular the system or components thereof as shown in any of the preceding figures.

Within the method, a plurality of stations 100, 200, 300 is provided, each station including at least one predefined object 101, 201, 301 to be handled by a test person, wherein a predefined manner of handling the predefined object is defined for each predefined object, and each station comprising a localization arrangement 110, 210, 310.

A test person is equipped with a mobile kit 500, which includes three wearable inertial sensors 501, 502, 503 to be worn by the test person and which have a memory configured for recording and storing sensor data indicative of a movement of the test person.

The mobile kit 500 further includes a mobile electronic device 550/560 with a localization device configured for interaction with the localization arrangement 110, 210, 310 of each station, and with an output device for providing information to the test person.

An input device 900 is provided to a supervisor.

A server 1000 is provided, which is communicatively connected or connectible to the input device 900, to the mobile electronic device 550/560, and to the inertial sensors 501, 502, 503.

In a first step S1 which is usually performed after taking the preparations above, the supervisor selects, on the input device 900, a subset of the stations 100, 200, 300 and a subset of specific objects 101, 201, 203 of those stations. For each selected object, a manner in which it shall be handled is predefined.

In a next step S2, this selection is passed on to the server 1000, which, in a next step S3, provides this list of stations and objects and manners in which they shall be handled to the mobile electronic device 550/560.

Based thereon, in a subsequent step S5, the mobile electronic device 550/560 then provides instructions to the test person, to move to the specific pre-selected stations and to handle the specific pre-selected predefined objects of these stations in their respective predefined manners. In the example given here, the test person receives an instruction to proceed towards station 100, and to handle object 101 in the predefined manner associated with it.

In a step S4, before or after the instructions are provided to the test subject by way of the mobile electronic device 550/560, the supervisor starts observing the test subject, and starts to take note of his movements.

The test person, having received the above-mentioned instructions, approaches station 100, and, when they arrive at the station 100, in step S6, the localization device of the mobile electronic device 550/560 interacts with the localization arrangement 110 of station 100, which indicates proximity of the test person to station 100. The interaction between the localization device 510 and the localization arrangement is thus detected and based thereon, it can be determined that the localization device 510 and thus the test person is in proximity of the localization arrangement 110 of the specific station 100.

In response to the detected proximity of step S6, step S7 is executed. Therein the mobile electronic device 550/560 communicates with the inertial sensors 501, 502, 503, sending information that is indicative of station 100, and, in a possible example, indicative of the specific object 101, and which signal is saved to the memory of the inertial sensors 501, 502, 503. The inertial sensors 501 502, 503 then start recording their sensor data, associated with the received information, which makes it possible to associate the recorded sensor data with station 100 and object 101.

In step S8, the test person proceeds to handle the object 101 of station 100 in the predefined manner, while recording of the sensor data is still active. At the same time, in step 9, the sensors monitor the movements that are being performed by the test person and store the corresponding sensor data in their memory. This sensor data can then be linked to object 101, because the sensors also hold the information indicative of station 100 and object 101 in their memory.

After the test person has finished the assigned tasks at station 100, a further interaction between the localization device of the mobile electronic device 550/560 and the localization arrangement 110 of the station 100 takes place in step 510, and in step S11, the mobile electronic device communicates termination of the station 100 to the inertial sensors, and the sensors store an indication of the termination to their memory and/or stop recording.

Steps S5-S10 are repeated, mutatis mutandis, for the further stations 200, 300 selected by the supervisor.

After the test person has gone through all of the preselected stations and handled all of the preselected objects, the information enabling identification of each specific station and the sensor data associable with it are uploaded to the server 1000, in step 512.

In step 13, input options are given to the supervisor by way of the input device 900, to allow the supervisor to input an assessment of a quality of predefined aspects of one or more movements carried out by the test person when handling the predefined objects in the respective predefined manners.

The input options include input options for one or more of assessing a movement or a posture of a shoulder of the test person, assessing a movement or a posture of a back of the test person, assessing a movement or a posture of a leg or of legs of the test person, assessing a movement or a posture of an arm or of arms of the test person, assessing a movement or a posture of a hand or of hands of the test person.

In step S14, the supervisor's input is transmitted from the input device 900 to the server 1000.

In step S15, the server calculates an ergonomic score based on the information received from the inertial sensors. From the sensor data, the server 1000 identifies for instance that one or more of the following actions have been performed by the test person: walking, standing, driving a vehicle, handling a hand cart, handling an object using one hand, handling an object using two hands, lifting an object, holding an object, carrying an object, bending a back of the test person, torsion of the back of the test person, handling an object in front of a body of the test person, handling an object above a shoulder of the test person. In order to identify these types of movement, the server employs a machine learning algorithm. The server also evaluates whether these actions have been performed in an ergonomically favorable manner, or in a potentially harmful manner.

The server 1000 also retrieves information on each specific object that has been handled by the test person, such as the object's size, shape, and weight. This way, the server can determine whether any of these characteristics are particularly problematic for the test person.

The server also retrieves information on how each object was supposed to be handled. This information may be compared to the movements actually performed by the test person, according to the sensor data, and a potential mismatch may be determined.

Overall, employing a machine learning algorithm, the server processes the sensor data, along with the information on each specific object, and the information on the predefined manner of handling each specific object, in order to evaluate a quality of movements carried out by the test person when handling each specific object in the predefined manner. The server may also include the input given by the supervisor into the evaluation of the quality of movements.

The whole process may be repeated, for example after a training has been completed under the supervision of the supervisor and/or after some weeks or months, giving the test person the opportunity to improve their movements.

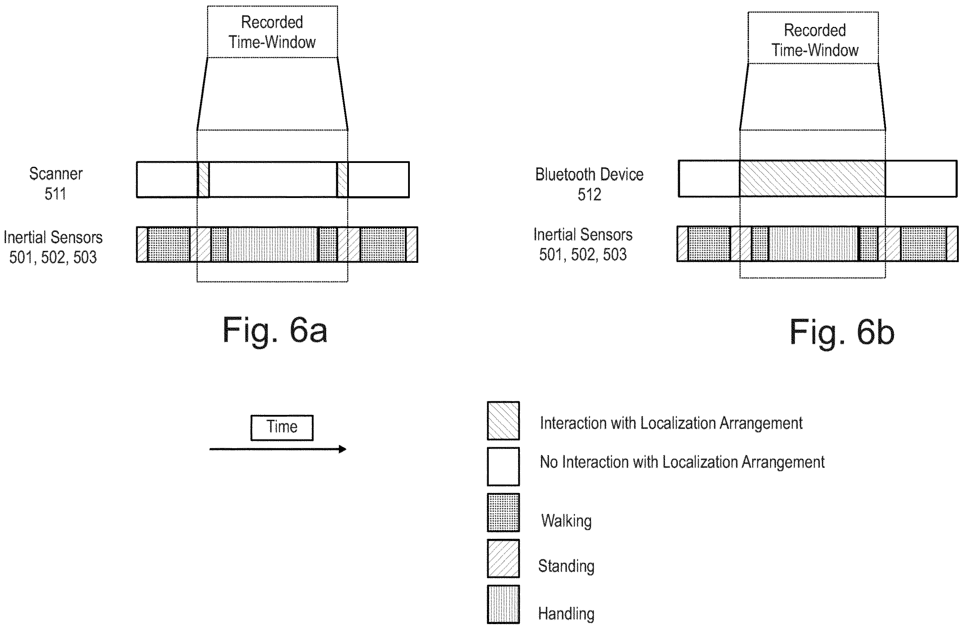

Figs. 6a and 6b illustrate association of sensor data of the wearable inertial sensors with data indicative of a proximity of a mobile electronic device to a specific station. The processes discussed in Figs. 6a and 6b represent specific ways of carrying out steps S6-S11 of the method of Figure 5.

Figures 6a and 6b schematically show a chronological sequence of operations within the localization device 510 (in the form of a scanner 511 or in the form of a Bluetooth device 512 (Second Wireless Communication Device), respectively) and within the inertial sensors 501, 502, 503.

For the scanner 511 and the Bluetooth device 512, there are two different states: Interaction with the localization arrangement of a station / no interaction with any localization arrangement.

The inertial sensors 501, 502, 503 are configured to acquire sensor data indicative of different actions performed by the test person, namely walking, standing, and handling, wherein handling may include driving a vehicle, handling a hand cart, handling an object using one hand, handling an object using two hands, lifting an object, holding an object, carrying an object, bending a back of the test person, torsion of the back of the test person, handling an object in front of a body of the test person, handling an object above a shoulder of the test person. These types of movement are identifiable within the method and system shown herein, based on the acquired sensor data.

The inertial sensors 501, 502, 503 are connected to a memory. The inertial sensors 501, 502, 503, the scanner 511 or Bluetooth device 512 and the memory are connected in such a manner that saving the sensor data to the memory is initiated and terminated based on an interaction of the scanner 511 or Bluetooth device 512 with the localization arrangement of a station. These will now be explained in more detail for the two types of localization devices.

Fig. 6a relates to a system where detecting proximity to a station is based on scanning a scannable code by way of the scanner 511 or the localization device. In this case, interaction between the localization device and the localization arrangement is therefore based on a specific action taken by the test person at a specific point in time ("test person scans code").

As can be seen from Figure 6a, the inertial sensors 501, 502, 503 are active and they acquire data indicative of standing, walking, and standing again.

While the test person is standing, they scan a scannable code of a given station by way of the scanner 511. This triggers an interaction between the scanner 511 and the inertial sensors 501, 502, 503, which receive an indication of the specific station at which the scanned code is located, and which save this indication to the memory, and start recording sensor data in a manner associated with the indication of the specific station. Consequently, the wearable inertial sensors record the relevant sensor data in a manner associable with the specific station, the sensor data being indicative of the test person's standing, walking, handling, walking, and standing again. Handling may include handling one or more objects at the given station. Therein, specific actions in relation to handling the one or more objects are identifiable from the sensor data, and it may be extracted from the sensor data that the test person has finished handling a first object, and has moved on to the next object etc. This way, handling of each object can later be linked to the supplementary information on each object, such as its size, shape, weight, and the predefined specific way in which it should be handled.

As can be seen from Figure 6b, a second interaction between the scanner 511 and the localization takes place after the test person has finished handling the object(s). I.e., the test person once again scans the code (or a different code) provided at the station. The system takes this as an indication that the station is finished, and a signal is submitted to the inertial sensors 501, 502, 503 so that they can stop recording. The stored data of the recorded time window can be associated to the specific station and its objects later on.

The process of Fig. 6b differs from the one of Fig. 6b in that the proximity is detected with the help of a Bluetooth beacon, and no specific interaction or action by the test person is required. It is sufficient that the test person enters into the proximity of the station while wearing or carrying the Bluetooth device 512. As soon as proximity is determined with the help of the localization arrangement in the form of the Bluetooth beacon, information allowing identification of the station is transmitted to the memory, and the inertial sensors 501, 502, 503 start recording their sensor data in a manner associated or associable therewith. After the test person is finished with handling one or more objects at the station, they move away from the station and thus out of the range of the Bluetooth beacon. Terminating the presence within the range of the Bluetooth beacon forms another interaction with the localization arrangement of the station, which indicates that the station has been completed, and subsequent to this, the sensors are instructed to stop recording.

### List of reference numerals

- 100, 200, 300: Stations
- 101, 102, 103: Predefined Objects
- 201, 202, 203, 204, 205: Predefined Objects
- 301, 302, 303: Predefined Objects
- 110, 210, 310: Localization Arrangement
- 111, 211: Scannable Code
- 112: First Wireless Communication Device
- 150, 250, 350: Shelf/Rack/Stack
- 160: Hand Cart

- 500: Mobile Kit
- 501, 502, 503: Wearable Inertial Sensors
- 510: Localization Device
- 511: Scanner
- 512: Second Wireless Communication Device
- 550: Portable Data Terminal
- 560: Smartphone
- 570: Output Device (Screen)
- 580: Communication System

- 600: Docking Station
- 610: Server Communication (Wireless)

- 900: Input Device
- 910: Second Server Communication (Wireless)

- 1000: Server

## Claims

1. A system for ergonomic training, comprising:
- a server (1000),
- a plurality of stations (100, 200, 300), each of the stations (100, 200, 300) including at least one predefined object (101, 102, 201, 202, 301, 302) to be handled by a test person, wherein a predefined manner of handling the predefined object (101, 102, 201, 202, 301, 302) is defined for each predefined object (101, 102, 201, 202, 301, 302), and each station (100, 200, 300) comprising a localization arrangement (110, 210, 310),
- a mobile kit (500) to be carried by a test person, including at least three wearable inertial sensors (501, 502, 503) to be worn by the test person and configured for recording and storing sensor data indicative of a movement of the test person, the mobile kit (500) further including a localization device (510) configured for interaction with the localization arrangement (110, 210, 310) of each station (100, 200, 300),
wherein the system is configured to determine that the localization device (510) is in proximity of the localization arrangement (110, 210, 310) of a specific station (100, 200, 300), based on an interaction between the localization device (510) and the localization arrangement (110, 210, 310) of the specific station,
the system further being configured for storing information enabling identification of the specific station (100, 200, 300), in response to determining the proximity between the localization arrangement (110, 210, 310) of the specific station and the localization device (510), and for storing subsequently recorded sensor data in a manner associable with the information enabling identification of the specific station (100, 200, 300),
wherein the server (1000) is configured for receiving the information enabling identification of the specific station (100, 200, 300) and the sensor data associable with it,
wherein the server (1000) is configured for retrieving information on the at least one predefined object (101, 102, 201, 202, 301, 302) and on the predefined manner of handling the at least one predefined object (101, 102, 201, 202, 301, 302) for the specific station,
wherein the server (1000) comprises a machine learning algorithm configured to process the sensor data, along with the information on the at least one predefined object (101, 102, 201, 202, 301, 302) and on the predefined manner of handling the at least one predefined object (101, 102, 201, 202, 301, 302), in order to evaluate a quality of movements carried out by the test person when handling the at least one predefined object (101, 102, 201, 202, 301, 302) in the predefined manner.

2. The system of claim 1, wherein evaluation of the quality of the movements includes identification of one or more of the following actions performed by the test person, based on the sensor data: walking, standing, driving a vehicle, handling a hand cart (160), handling an object using one hand, handling an object using two hands, lifting an object, holding an object, carrying an object, bending a back of the test person, torsion of the back of the test person, handling an object in front of a body of the test person, handling an object above a shoulder of the test person.

3. The system of any of the preceding claims, wherein the at least three wearable inertial sensors (501, 502, 503) include a first wearable inertial sensor (501) that is included in a waist- or hip-belt and a second wearable inertial sensor (502) that is included in a wrist band and a third wearable inertial sensor (503) that is included in a further wrist band.

4. The system of any of the preceding claims, wherein the mobile kit (500) comprises a mobile electronic device (550, 560), such as a Portable Data Terminal (550) or a smartphone (560) or a portable computer, wherein the mobile electronic device preferably includes the localization device (510) and/or wherein the mobile electronic device preferably comprises an output device (570) for providing instructions to the test person.

5. The system of claim 4, wherein the mobile kit (500) comprises a communication system (580) for communication between the mobile electronic device (550, 560) and the at least three wearable inertial sensors (501, 502, 503).

6. The system of any of the preceding claims, wherein the localization arrangement (110, 210, 310) includes a first wireless communication device (112), in particular a beacon, and the localization device (510) comprises a second wireless communication device (512), configured for interaction with the first wireless communication device (112) and/or wherein the localization arrangement (110, 210, 310) comprises a scannable code (111, 211), in particular a barcode or a QR-Code, and the localization device (510) comprises a scanner (511) for scanning the scannable code (111, 211).

7. The system of any of the preceding claims, wherein the mobile kit (500) includes at least one memory for storing the information enabling identification of the specific station and the sensor data associable with it, the system preferably comprising a docking station (600) for at least some of the components of the mobile kit (500), the docking station (600) being connected to the server (1000) and enabling upload of the information enabling identification of the specific station and the sensor data associable with it from the memory to the server (1000).

8. The system of any of the preceding claims, wherein the wearable inertial sensors (501, 502, 503) include an accelerometer and/or a gyroscope and/or a magnetometer and/or wherein a recording rate of the sensors is at least 33 Hz, for example the recording rate being 100 Hz.

9. The system of any of the preceding claims, wherein the plurality of stations (100, 200, 300) comprises at least 5 stations or at least 20 stations, for instance 30 stations, and/or wherein at each station of the plurality of stations (100, 200, 300) at least two or at least three predefined objects are provided and/or wherein the plurality of stations (100, 200, 300) include predefined objects (101, 102, 201, 202, 301, 302) that differ from each other in weight and/or size, wherein the server (1000) is configured for retrieving information on weight and/or size of each predefined object (101, 102, 201, 202, 301, 302) and for including this information in the evaluation of the quality of movements carried out by the test person when handling the respective predefined object (101, 102, 201, 202, 301, 302) in the predefined manner.

10. The system of any of the preceding claims, wherein the predefined manner of handling the predefined object (101, 102, 201, 202, 301, 302) includes, at at least some of the stations (100, 200, 300), one or more of picking the object (101, 102, 201, 202, 301, 302), lifting up the object (101, 102, 201, 202, 301, 302), removing the object (101, 102, 201, 202, 301, 302) from a shelf or rack (250, 350), putting down the object (101, 102, 201, 202, 301, 302), inserting the object (101, 102, 201, 202, 301, 302) into a shelf or rack (150, 250), carrying the object (101, 102, 201, 202, 301, 302) over a distance, moving the object (101, 102, 201, 202, 301, 302) by way of a hand cart (160).

11. The system of any of the preceding claims, further including an input device (900) for a supervisor, configured for providing one or more input options to allow the supervisor to input an assessment of a quality of predefined aspects of one or more movements carried out by the test person when handling the predefined object (101, 102, 201, 202, 301, 302) in the predefined manner,
wherein the server (1000) is configured for receiving the input by the supervisor, and for including said input by the supervisor into the evaluation of the quality of movements carried out by the test person when handling the object (101, 102, 201, 202, 301, 302) in the predefined manner.

12. The system of claim 11, wherein the one or more input options include an input option for one or more of
assessing a movement or a posture of a shoulder of the test person,
assessing a movement or a posture of a back of the test person,
assessing a movement or a posture of a leg or of legs of the test person,
assessing a movement or a posture of an arm or of arms of the test person,
assessing a movement or a posture of a hand or of hands of the test person.

13. The system of claim 11 or 12, wherein the input device (900) for the supervisor provides an input option for selecting a subset of the plurality of stations (100, 200, 300) by the supervisor and/or for selecting a subset of predefined objects of a given station by the supervisor and/or wherein the input device (900) for the supervisor is connected to the internet, and the input options for the supervisor are provided in a web-interface.

14. A method for ergonomic training, comprising:
providing a plurality of stations (100, 200, 300), each station including at least one predefined object (101, 102, 201, 202, 301, 302) to be handled by a test person, wherein a predefined manner of handling the predefined object (101, 102, 201, 202, 301, 302) is defined for each predefined object (101, 102, 201, 202, 301, 302), and each station comprising a localization arrangement (110, 210, 310);
equipping a test person with a mobile kit (500), including at least three wearable inertial sensors (501, 502, 503) to be worn by the test person and configured for recording and storing sensor data indicative of a movement of the test person, the mobile kit (500) further including a localization device configured for interaction with the localization arrangement (110, 210, 310) of each station;
providing instructions to the test person to handle at least a subset of the at least one predefined object (101, 102, 201, 202, 301, 302) of at least a subset of the plurality of stations (100, 200, 300) in the respective predefined manner;
wherein the test person moves consecutively to each of the stations (100, 200, 300) of the subset of the plurality of stations (100, 200, 300), and handles the subset of the at least one predefined object (101, 102, 201, 202, 301, 302) at each of these stations in the respective predefined manner, and the following steps are executed:
- detecting an interaction between the localization device (510) and the localization arrangement (110, 210, 310) of a specific station, and, based on the detected interaction, determining that the localization device (510) is in proximity of the localization arrangement (110, 210, 310) of the specific station,
- storing information enabling identification of the specific station, in response to determining the proximity between the localization arrangement (110, 210, 310) of the specific station and the localization device (510), and subsequently recording sensor data in a manner associable with the information enabling identification of the specific station,
- uploading the information enabling identification of the specific station and the sensor data associable with it to a server (1000),
- retrieving information on each specific object of the subset of the at least one predefined object (101, 102, 201, 202, 301, 302), and on the predefined manner of handling each specific object,
- employing a machine learning algorithm, for processing the sensor data, along with the information on each specific object, and on the predefined manner of handling each specific object, in order to evaluate a quality of movements carried out by the test person when handling each specific object in the predefined manner.

15. The method of claim 14, including
providing an input device (900) to a supervisor,
providing, on the input device (900), one or more input options to allow the supervisor to input an assessment of a quality of predefined aspects of one or more movements carried out by the test person when handling the predefined object (101, 102, 201, 202, 301, 302) in the predefined manner,
transmitting the supervisor's input to the server (1000), and including said input into the evaluation of the quality of movements carried out by the test person when handling the object (101, 102, 201, 202, 301, 302) in the predefined manner.
